(19) **Europäisches Patentamt / European Patent Office / Office européen des brevets**

(11) **EP 4 536 312 B1**

(12) # EUROPEAN PATENT SPECIFICATION

(45) Date of publication and mention
of the grant of the patent:
**18.02.2026  Bulletin 2026/08**

(21) Application number: **23730611.3**

(22) Date of filing: **05.06.2023**

(51) International Patent Classification (IPC):
**A61M 1/16** *(2006.01)*    **A61M 1/36** *(2006.01)*
**A61M 16/00** *(2006.01)*   **A61M 16/08** *(2006.01)*
**A61M 16/10** *(2006.01)*   **A61M 16/12** *(2006.01)*
**A61B 5/083** *(2006.01)*   **G01N 33/00** *(2006.01)*
**G01N 33/497** *(2006.01)*

(52) Cooperative Patent Classification (CPC):
**A61M 1/1698; A61B 5/0836; A61M 1/3666;
A61M 16/024; A61M 16/1005; G01N 33/004;
G01N 33/497;** A61M 16/0808; A61M 16/12;
A61M 16/161; A61M 2016/0027; A61M 2016/0033;
A61M 2016/1025; A61M 2016/103;
A61M 2016/1035;                                (Cont.)

(86) International application number:
**PCT/SE2023/050562**

(87) International publication number:
**WO 2023/239283 (14.12.2023 Gazette 2023/50)**

(54) **MULTISAMPLING SIDESTREAM GAS ANALYSER FOR GAS EXCHANGE ANALYSIS**

MEHRPROBEN-SEITENSTROM-GASANALYSATOR ZUR GASAUSTAUSCHANALYSE

ANALYSEUR DE GAZ À SOUTIRAGE LATÉRAL ET ÉCHANTILLONNAGE MULTIPLE POUR
ANALYSE D'ÉCHANGE GAZEUX

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **09.06.2022   SE 2250695**

(43) Date of publication of application:
**16.04.2025   Bulletin 2025/16**

(73) Proprietor: **Maquet Critical Care AB
171 06 Solna (SE)**

(72) Inventors:
• **LARSSON, Åke**
  **175 64 Järfälla (SE)**
• **LONCAR, Mario**
  **Ekerö 178 39 (SE)**

(74) Representative: **Zacco Sweden AB
P.O. Box 5581
Löjtnantsgatan 21
114 85 Stockholm (SE)**

(56) References cited:
**US-A1- 2014 216 252     US-A1- 2020 359 935
US-A1- 2021 353 892**

EP 4 536 312 B1

(52) Cooperative Patent Classification (CPC): (Cont.)
A61M 2202/0208; A61M 2202/0225; A61M 2205/05;
A61M 2205/3313; A61M 2205/3331;
A61M 2205/3334; A61M 2205/3368;
A61M 2205/502; A61M 2205/70; A61M 2205/7527;
A61M 2230/432; A61M 2230/435; G01N 33/0006

**Description**

TECHNICAL FIELD

The present disclosure relates to a system for analysing gas concentrations before and after a point of gas exchange and, in particular, to a sidestream gas analyser for determining a measure of gas exchange at the point of gas exchange.

BACKGROUND

When measuring differential gas concentrations, for example before and after something that removes gas from a gas mixture, differences in gas analyser characteristics and calibration may significantly reduce measurement accuracy.
For example, when measuring fractions of oxygen (O2) and/or carbon dioxide (CO2) in a sweep gas flow of an oxygenator for Extracorporeal Membrane Oxygenation (ECMO) treatment of a patient, differences in characteristics between a pre-oxygenator gas analyser and a post-oxygenator gas analyser inevitably introduce errors in determination of the O2 and/or CO2 gas exchange over the oxygenator membrane.
Likewise, when measuring fractions of O2 and CO2 in an inspiratory line and an expiratory line of a mechanical ventilator for respiratory treatment of a patient, differences in sensor characteristics introduce errors in determination of O2 uptake and CO2 removal in the lungs of the patient.
US 2021/353892 discloses a system for ventilating and oxygenating of a patient, wherein separate gas analysis units are provided for process gas analysis and blood gas analysis.
US 2020/359935 discloses a ventilation system, comprising a gas analyser connected to the inspiratory line and the expiratory line.
US 2014/216252 discloses an oxygenation system comprising carbon dioxide sensors on the sweep gas inlet line and the sweep gas outlet line.

SUMMARY

It is an object of the present disclosure to address the above identified deficiencies of the prior art.
It is a specific object of the present disclosure to present a system for reliable determination of a measure of gas exchange occurring at a point of gas exchange, e.g., in an oxygenator of an ECMO device or in the lungs of a patient undergoing mechanical ventilation.
This and other objects, which will become apparent in view of the detailed description following hereinafter, are achieved in accordance with a first aspect of the present disclosure by a system for analysing gas concentrations before and after first and second points of gas exchange, as set forth in the appended claims.
The system comprises a gas inlet line for conveying a flow of an input gas mixture towards the point of gas exchange, a gas outlet line for conveying a flow of an output gas mixture away from the point of gas exchange, and a gas analyser that is connected to both the gas inlet line and the gas outline line and configured to sequentially receive and analyse gas samples from the gas inlet line and the gas outlet line in order to determine a presence of a specific gas in the input gas mixture and the output gas mixture.
By using the same gas analyser for analysing samples of both the input gas mixture and the output gas mixture, measurement errors due to inter-analyser differences, e.g. in terms of sensor characteristics and calibration, can be avoided. Furthermore, a compact and cost-efficient differential gas concentration analyser arrangement is achieved.
According to some embodiments, the system comprises at least one processor coupled to the gas analyser. The at least one processor may be configured to determine an input fraction of the specific gas in the input gas mixture based on the analysis by the gas analyser of the gas samples from the gas inlet line, determine an output fraction of the specific gas in the output gas mixture based on the analysis by the gas analyser of the gas samples from the gas outlet line, and determine, based on the input and output fractions of the specific gas, a measure of gas exchange of the specific gas at the point of gas exchange.
This allows a measure of gas exchange of the specific gas at the point of gas exchange to be provided as decision support to an operator, or as a control parameter for manual, semi-automatic or automatic control of a level of gas exchange at the point of gas exchange.
According to some embodiments, the at least one processor is configured to:

- receive a measurement of an input gas mixture flow rate ($\dot{V}_{in}$) of the input gas mixture in the gas inlet line,
- receive a measurement of an output gas mixture flow rate ($\dot{V}_{out}$) of the output gas mixture in the gas outlet line,
- determine, based on the input fraction of the specific gas and the input gas mixture flow rate, an input net flow of the specific gas in the gas inlet line,

- determine, based on the output fraction of the specific gas and the output gas mixture flow rate, a net flow of the specific gas in the gas outlet line, and
- determine a change in net flow of the specific gas at the point of gas exchange based on the input net flow and the output net flow of the specific gas, which change in net flow is indicative of a gas exchange of the specific gas at the point of gas exchange.

This allows the gas exchange to be quantified in terms of a net flow of the specific gas in either direction at the point of gas exchange. For example, in scenarios where the specific gas is carbon dioxide ($CO_2$) and the point of gas exchange is an oxygenator membrane of an ECMO device for providing ECMO treatment to a patient, the change in net flow of $CO_2$ corresponds to the net $CO_2$ exchange ($\dot{V}CO2_{net}$) over the membrane and so indicates actual $CO_2$ removal (or addition) in ml/min obtained by the ECMO device. This metric can be presented to an operator of the ECMO device, or be used as a control parameter in manual, semi-automatic or automatic control of a sweep gas flow rate and/or an addition of $CO_2$ to the sweep gas flow to meet a set target value for the net $CO_2$ exchange. In this scenario, the proposed technique is also advantageous in that it presents a purely gas based approach for determining actual $CO_2$ removal by the ECMO device, without the need for blood gas analysis.

According to some embodiments, the system comprises a gas sample conditioner for removal or reduction of water vapour in the gas samples from the gas inlet line and/or the gas samples from the gas outlet line, prior to analysis of the gas samples by the gas analyser. In this case, the at least one processor may be configured to determine the input fraction of the specific gas in the input gas mixture based on a fraction of the specific gas in an analysed input gas sample and an estimated removal of water vapour ($\Delta FH2O_{in}$) from the input gas sample by the gas sample conditioner, and/or to determine the output fraction of the specific gas in the output gas mixture based on a fraction of the specific gas in an analysed output gas sample and an estimated removal of water vapour [$\Delta FH2O_{out}$] from the output gas sample by the gas sample conditioner.

Reducing the water vapour content in the gas samples before the gas samples enters the gas analyser is advantageous in that it prevents or reduces condensation of water vapour in the gas analyser. However, it has the undesired effect of altering the composition of the gas samples and thus creating a discrepancy between the fraction of the specific gas in the gas samples measured upon and the actual fraction of the specific gas in the gas inlet or outlet line from which the gas samples were withdrawn. By estimating a removal of water vapour by the gas sample conditioner and taking the estimated removal of water vapour into account, a compensated fraction of the specific gas corresponding to the actual fraction of the specific gas in the gas inlet or outlet line can be determined from the measured fraction of the specific gas in the gas samples.

According to some embodiments, the gas analyser is connected also to a calibration gas source for delivery of a calibration gas, the gas analyser being configured to sequentially receive and analyse gas samples from the gas inlet line, the gas outlet line, and the calibration gas source.

This has the effect that the accuracy of the gas analyser can be periodically or intermittently verified, and that the gas analyser can be periodically or intermittently calibrated, thus preventing drift and increasing gas analysis accuracy.

According to some embodiments, the specific gas is selected from the group consisting of carbon dioxide [$CO_2$] and oxygen [$O_2$].

According to some embodiments, the gas analyser comprises a first sensor for measuring a fraction of $CO_2$ (FCO2in) in the input gas mixture and a fraction of $CO_2$ (FCO2out]) in the output gas mixture, and a second sensor for measuring a fraction of $O_2$ (FO2in) in the input gas mixture and a fraction of $O_2$ [FO2out] in the output gas mixture.

According to some embodiments, the first sensor is a non-dispersive infrared (NDIR) $CO_2$ sensor and the second sensor is a paramagnetic or electrochemical $O_2$ sensor.

According to some embodiments of the disclosure, the system comprises a device (ECMO device) for extracorporeal blood gas exchange, the device comprising:

- an oxygenator including a membrane acting as a gas-liquid barrier enabling $CO_2$ transfer between a bloodstream and a sweep gas flow through the oxygenator, wherein the membrane constitutes the point of gas exchange,
- a sweep gas inlet line for conveying an input sweep gas towards the membrane, and a sweep gas outlet line for conveying an output sweep gas away from the membrane,

wherein the gas analyser is connected to both the sweep gas inlet line and the sweep gas outline line and configured to sequentially receive and analyse gas samples from the sweep gas inlet line and the sweep gas outlet line, in order to analyse a presence of the specific gas in the input sweep gas flow and in the output sweep gas flow.

In this application, the above mentioned advantages of the gas analyser result in a very precise analysis of the gas exchange of, e.g., $CO_2$ and/or $O_2$ taking place over the oxygenator membrane, while at the same time providing for a compact and cost efficient oxygenator configuration.

According to some embodiments of the disclosure, the system comprises a mechanical ventilator for mechanically ventilating a patient through the supply of breathing gas to the lungs of the patient, wherein the lungs of the patient constitutes the point of gas exchange, the ventilator comprising:

- an inspiratory line for conveying a flow of breathing gas towards the patient, and an expiratory line for conveying a flow of exhalation gas away from the patient,

wherein the gas analyser is connected to both the inspiratory line and the expiratory line and configured to sequentially receive and analyse gas samples from the inspiratory line and the expiratory line, in order to analyse a presence of the specific gas in the flow of breathing gas and in the flow of exhalation gas.

In this application, the above mentioned advantages of the gas analyser result in a very precise analysis of the gas exchange of, e.g., CO2 and/or O2 taking place in the lungs of the patient, while at the same time providing for a compact and cost efficient ventilator configuration.

According to the invention, the system is a combined system, herein referred to as an ECMO-vent system, which comprises both an ECMO device and a mechanical ventilator as set forth above, wherein the gas analyser is connected to both the sweep gas inlet line and the sweep gas outline line of the ECMO device, and to the inspiratory line and the expiratory line of the ventilator. The gas analyser is configured to sequentially receive and analyse gas samples from the sweep gas inlet line, the sweep gas outlet line, the inspiratory line, and the expiratory line, whereas the at least one processor is configured to determine a measure of gas exchange of the specific gas in the oxygenator based on the analysis of the gas samples from the sweep gas inlet line and the sweep gas outlet line, and to determine a measure of gas exchange of the specific gas in the lungs of the patient based on the analysis of the gas samples from the inspiratory line and the expiratory line.

Consequently, the system comprises

a device for extracorporeal blood gas exchange, comprising:

- an oxygenator including a membrane acting as a gas-liquid barrier enabling CO2 transfer between a bloodstream and a sweep gas flow through the oxygenator, wherein the membrane constitutes a first point of gas exchange, and
- a sweep gas inlet line for conveying an input sweep gas towards the membrane, and a sweep gas outlet line for conveying an output sweep gas away from the membrane, and

a mechanical ventilator for mechanically ventilating a patient through the supply of breathing gas to the lungs of the patient, wherein the lungs of the patient constitutes a second point of gas exchange, the ventilator comprising:

- an inspiratory line for conveying a flow of breathing gas towards the patient, and an expiratory line for conveying a flow of exhalation gas away from the patient,

wherein the gas analyser is connected to both the sweep gas inlet line, the sweep gas outline line, the inspiratory line, and the expiratory line and configured to sequentially receive and analyse gas samples from the sweep gas inlet line, the sweep gas outlet line, the inspiratory line, and the expiratory line, the gas analyser comprising at least one processor configured to determine a measure of gas exchange of the specific gas at the first point of gas exchange based on the analysis of the gas samples from the sweep gas inlet line and the sweep gas outlet line, and to determine a measure of gas exchange of the specific gas at the second point of gas exchange based on the analysis of the gas samples from the inspiratory line and the expiratory line.

Besides offering a compact and cost efficient design of the ECMO-vent system, the use of a common gas analyser eliminates measurement inaccuracies normally caused by differences in sensor characteristics and calibration, which improves gas analysis and gives a more detailed view of the total gas exchange achieved by the ECMO treatment and the respiratory treatment. For example, when the gas analyser is used for CO2 measurements, the improved accuracy in CO2 determination provide for a more detailed understanding of the combined effect of CO2 removal achieved by the ECMO device and the ventilator, which allows for improved and more efficient use and control of the ECMO device and the ventilator and, ultimately, a safer and more efficient treatment of the patient.

According to some embodiments, the at least one processor is configured to:

- determine an input fraction of the specific gas in the input sweep gas based on the analysis by the gas analyser of the gas samples from the sweep gas inlet line,
- determine an output fraction of the specific gas in the output sweep gas based on the analysis by the gas analyser of the gas samples from the sweep gas outlet line, and

- determine, based on the input and output fractions of the specific gas, a measure of gas exchange of the specific gas at the first point of gas exchange.

According to some embodiments, the at least one processor is configured to:

- receive a measurement of a flow rate of the input sweep gas in the sweep gas inlet line,
- receive a measurement of a flow rate of the output sweep gas in the sweep gas outlet line,
- determine, based on the input fraction of the specific gas in the input sweep gas and the flow rate of the input sweep gas, an input net flow of the specific gas in the sweep gas inlet line,
- determine, based on the output fraction of the specific gas in the output sweep gas and the flow rate of the output sweep gas, a net flow of the specific gas in the sweep gas outlet line, and
- determine a change in net flow of the specific gas at the first point of gas exchange based on the input net flow and the output net flow of the specific gas, which change in net flow is indicative of a gas exchange of the specific gas at the first point of gas exchange.

According to some embodiments, the at least one processor is configured to:

- determine an input fraction of the specific gas in the breathing gas based on the analysis by the gas analyser of the gas samples from the inspiratory line,
- determine an output fraction of the specific gas in the exhalation gas based on the analysis by the gas analyser of the gas samples from the expiratory line, and
- determine, based on the input and output fractions of the specific gas, a measure of gas exchange of the specific gas at the second point of gas exchange.

According to some embodiments, the at least one processor is configured to:

- receive a measurement of a flow rate of the breathing gas in the inspiratory line,
- receive a measurement of a flow rate of the exhalation gas in the expiratory line,
- determine, based on the input fraction of the specific gas in the breathing gas and the flow rate of the breathing gas, an input net flow of the specific gas in the inspiratory line,
- determine, based on the output fraction of the specific gas in the exhalation gas and the flow rate of the exhalation gas, a net flow of the specific gas in the expiratory line, and
- determine a change in net flow of the specific gas at the first point of gas exchange based on the input net flow and the output net flow of the specific gas, which change in net flow is indicative of a gas exchange of the specific gas at the second point of gas exchange.

According to some embodiments, the system comprises a gas sample conditioner for removal of water vapour in the gas samples from the sweep gas inlet line and/or the gas samples from the sweep gas outlet line, prior to analysis of the gas samples by the gas analyser, the at least one processor being configured to:

- determine the input fraction of the specific gas in the input sweep gas based on a fraction of the specific gas in an analysed input sweep gas sample and an estimated removal of water vapour from the input sweep gas sample by the gas sample conditioner, and/or
- determine the output fraction of the specific gas in the output sweep gas based on a fraction of the specific gas in an analysed output sweep gas sample and an estimated removal of water vapour from the output sweep gas sample by the gas sample conditioner.

According to some embodiments, the system comprises a gas sample conditioner for removal of water vapour in the gas samples from the inspiratory line and/or the gas samples from the expiratory line, prior to analysis of the gas samples by the gas analyser), the at least one processor being configured to:

- determine the input fraction of the specific gas in the breathing gas based on a fraction of the specific gas in an analysed breathing gas sample and an estimated removal of water vapour from the breathing gas sample by the gas sample conditioner, and/or
- determine the output fraction of the specific gas in the exhalation gas based on a fraction of the specific gas in an analysed exhalation gas sample and an estimated removal of water vapour from the exhalation gas sample by the gas sample conditioner.

According to some embodiments, the gas analyser is connected also to a calibration gas source for delivery of a calibration gas, the gas analyser being configured to sequentially receive and analyse gas samples from the sweep gas inlet line, the sweep gas outlet line, the inspiratory line, the expiratory line, and the calibration gas source.

According to some embodiments, the system is configured to display gas exchange information on a monitor of the system, the gas exchange information comprising information related to the measure of gas exchange of the specific gas at the first point of gas exchange, information related to the measure of gas exchange of the specific gas at the second point of gas exchange, and/or information related to a measure of total gas exchange of the specific gas at the first and the second points of gas exchange..

This has the effect of providing a clinician with a visual overview of the gas exchange taking place in the oxygenator membrane of the ECMO device and in the lungs of the patient, thus facilitating use and control of the ECMO device and the ventilator. The measure of total gas exchange may also assist the clinician in the decision on whether to intensify or decrease the intensity of the treatment provided by any or both of the ECMO device or the ventilator.

More advantageous features of the system will be described in the detailed description following hereinafter.

BRIEF DESCRIPTION OF THE DRAWINGS

The present invention will become more fully understood from the detailed description provided hereinafter and the accompanying drawings, which are given by way of non-limiting illustration only. In the different drawings, same reference numerals correspond to the same element.

Fig. 1 illustrates an exemplary and non-limiting embodiment of a system for analysing gas concentrations before and after a point of gas exchange.

Fig. 2 illustrates an exemplary and non-limiting embodiment of a system for analysing gas concentrations before and after a point of gas exchange, wherein the system comprises an ECMO device for providing ECMO treatment to a patient.

Fig. 3 illustrates an exemplary and non-limiting embodiment of a system for analysing gas concentrations before and after a point of gas exchange, wherein the system is a combined system, herein referred to as an ECMO-vent system, comprising both an ECMO device for providing ECMO treatment to a patient and a mechanical ventilator for respiratory treatment of the patient.

Fig. 4 illustrates another exemplary and non-limiting embodiment of an ECMO-vent system, where a single gas analyser is used for analysing the gas exchange taking place in the ECMO device and in the lungs of the patient.

Fig. 5 is a flowchart illustrating an exemplary non-limiting embodiment of a method for controlling CO2 removal in and by the ECMO device.

DETAILED DESCRIPTION

The present disclosure relates to the field of measurements of gas concentrations in gas mixtures upstream and downstream of a point of gas exchange.

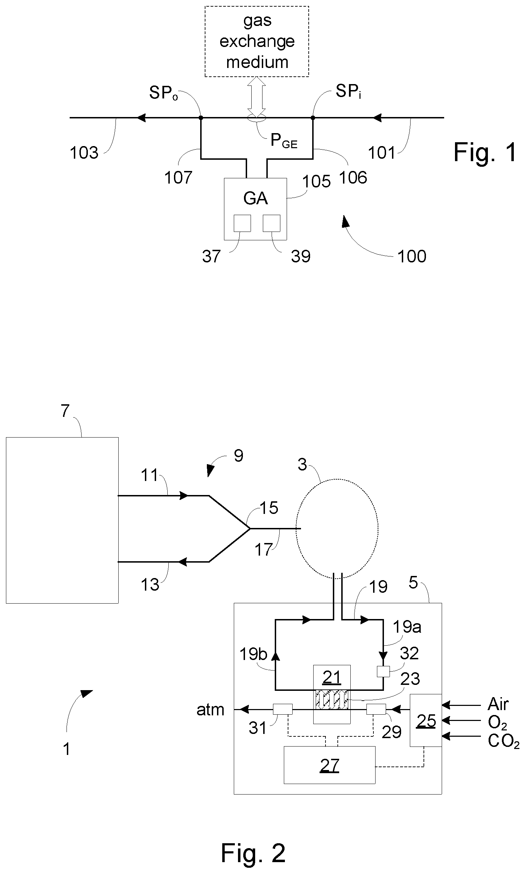

Fig. 1 illustrates a system 100 for analysing gas concentrations before and after a point of gas exchange, $P_{GE}$, according to an exemplary and non-limiting embodiment of the present disclosure.

**The system** 100 comprises a gas inlet line 101 for conveying a flow of an input gas mixture towards the point of gas exchange $P_{GE}$, and a gas outlet line 103 for conveying a flow of an output gas mixture away from the point of gas exchange $P_{GE}$. The system further comprises a gas analyser 105 that is connectable to both the gas inlet line 101 and the gas outline line 103 and configured to sequentially receive and analyse gas samples from the gas inlet line 101 and the gas outlet line 103 in order to determine a presence of a specific gas in the input gas mixture and the output gas mixture.

That the gas analyser 105 is configured to sequentially receive gas samples from the gas inlet line 101 and the gas outlet line 103 means that it is arranged to periodically or intermittently receive gas samples from the gas inlet line 101 and the gas outlet line 103. For example, the gas analyser 105 may be configured to sequentially withdraw and analyse gas samples from the gas inlet line 101 and the gas outlet line 103 such that a gas sample from the respective line is acquired and analysed every tenth second, or at any other time interval that may be determined based on, e.g., the performance of the gas analyser and/or the system architecture. After analysis of a gas sample, the gas analyser 105 is configured to return the gas sample to the inlet line or outlet line from which it was withdrawn.

The gas analyser 105 comprises a first sampling line 106 configured to be connected to the gas inlet line 101 to withdraw samples of the input gas mixture from an inlet sampling point $SP_i$ in the gas inlet line, and a second sampling line 107 configured to be connected to the gas outlet line 103 to withdraw samples of the output gas mixture from an outlet sampling point $SP_o$ in the gas outlet line. In some embodiments, the first and second sampling lines 106 and 107 may be separate gas lines connected to a respective gas inlet of the gas analyser 105. In other embodiments, the first and second sampling lines 106 and 107 may be connected to a common inlet of the gas analyser 105 via a common gas sampling line that is branched into the first and second sampling lines 106 and 107 for connection to the gas inlet line 101 and the gas outlet line 103, respectively.

The gas inlet line 101 could be a piece of tubing, a pipe, a hose or any other component that is located upstream of the point of gas exchange $P_{GE}$ and that serves **to convey** the input gas mixture towards the point of gas exchange $P_{GE}$, or to accommodate the input gas mixture on its way towards the point of gas exchange $P_{GE}$. Likewise, the gas outlet line 103 could be a piece of tubing, a pipe, a hose or any other component that is located downstream of the point of gas exchange $P_{GE}$ and that serves to convey the output gas mixture away from the point of gas exchange $P_{GE}$, or to accommodate the output gas mixture on its way from the point of gas exchange $P_{GE}$. The inlet sampling point $SP_i$ may be located anywhere along the gas inlet line 101 as long as there is no substantial change in composition of the input gas mixture between the inlet sampling point $SP_i$ and the point of gas exchange $P_{GE}$. Likewise, the outlet sampling point $SP_o$ may be located anywhere along the gas outlet line 103 as long as there is no substantial change in composition of the output gas mixture between the point of gas exchange $P_{GE}$ and the outlet sampling point $SP_o$.

The gas analyser 105 is a so called sidestream gas analyser, i.e. a gas analyser that is configured to withdraw gas samples from a main stream of gas to be analysed. In this case, where the gas analyser is configured to withdraw gas samples from more than one sampling point of the main gas stream, the gas analyser constitutes what may be referred to as a multisampling sidestream gas analyser.

The point of gas exchange $P_{GE}$ may be any point, place, location, physical unit or process at or in which a gas exchange takes place between the input gas mixture that is delivered to the point of gas exchange via the gas inlet line 101 and another medium, hereinafter referred to as a gas exchange medium. The gas exchange medium is typically a fluid, may it be liquid or gas, with which the input gas mixture in the gas inlet line 101 may interact through gas exchange to alter its composition. Non-limiting examples of a point of gas exchange in the context of the present disclosure include:

- an oxygenator of an ECMO device for ECMO treatment of a patient, where gas exchange between an extracorporeal blood flow of a patient and a sweep gas flow takes place over a membrane of the oxygenator, and where a sweep gas inlet line of the ECMO device constitutes said gas inlet line 101 and a sweep gas outlet line of the ECMO device constitutes said gas outlet line 103;
- a lung of a mechanically ventilated patient, where gas exchange between a pulmonary blood flow and lung gases takes place in the alveoli of the lung, and where an inspiratory line of the mechanical ventilator constitutes said gas inlet line 101 and an expiratory line of the mechanical ventilator constitutes said gas outlet line 103.

The gas analyser 105 is configured to determine a fraction or concentration of the specific gas in the inlet gas mixture and the outlet gas mixture. The specific gas may be any type of gas but the gas analyser 105 is particularly intended for medical applications where the specific gas is carbon dioxide ($CO_2$) or oxygen ($O_2$). In some embodiments, the gas analyser 105 may hence comprises a $CO_2$ sensor configured to measure the fraction of $CO_2$ in the gas samples from the gas inlet line 101 and the gas outlet line 103. In other embodiments, the gas analyser 105 may comprise an $O_2$ sensor configured to measure the fraction of $O_2$ in the gas samples from the gas inlet line 101 and the gas outlet line 103. In yet other embodiments, the gas analyser 105 may be comprise both a $CO_2$ sensor and an $O_2$ sensor for measuring both a fraction of $CO_2$ and a fraction of $O_2$ in the gas samples from the gas inlet line 101 and the gas outlet line 103. In some embodiments, the $CO_2$ sensor is a non-dispersive infrared (NDIR) $CO_2$ sensor. In some embodiments, the $O_2$ sensor is a paramagnetic or electrochemical $O_2$ sensor.

The gas analyser 105 may further comprise or be coupled to at least one processor 37 for processing and performing operations on the sensor data obtained by the one or more sensors of the gas analyser 105. The gas analyser 105 may also comprise or be coupled to at least one digital storage medium 39, such as a non-transitory hardware memory device, for storing sensor data and/or other data, as well as computer programs comprising computer-readable instructions that may be executed by the at least one processor 37 for performing various operations on the sensor data obtained by the gas analyser 105 and, optionally, on data obtained by other sensors or equipment (not shown) of the system 100.

The at least one processor 37 may configured to determine an input fraction of the specific gas in the input gas mixture based on the analysis by the gas analyser 105 of the gas samples from the gas inlet line 101, determine an output fraction of the specific gas in the output gas mixture based on the analysis by the gas analyser 105 of the gas samples from the gas outlet line 103, and determine, based on the input and output fractions of the specific gas, a measure of gas exchange of the specific gas at the point of gas exchange $P_{GE}$.

In some embodiments, the system 100 may comprise a first flow sensor (not shown) for measuring a flow rate ($\dot{V}_{in}$) of the input gas mixture in the gas inlet line 101, and a second flow sensor (not shown) for measuring a flow rate ($\dot{V}_{out}$)

of the output gas mixture in the gas outlet line 103. The at least one processor 37 may then be configured to receive measurements of $\dot{V}_{in}$ and $\dot{V}_{out}$ and to determine, based on $\dot{V}_{in}$ and the input fraction of the specific gas, an input net flow of the specific gas in the gas inlet line 101; determine, based on $\dot{V}_{out}$ and the output fraction of the specific gas, a net flow of the specific gas in the gas outlet line 103, and; determine a change in net flow of the specific gas at the point of gas exchange $P_{GE}$ based on the input net flow and the output net flow of the specific gas, which change in net flow is indicative of a net gas exchange of the specific gas at the point of gas exchange $P_{GE}$. For example, when the specific gas is $CO_2$, the at least one processor 37 is capable of determining a net $CO_2$ exchange ($\dot{V}CO_{2net}$) at the point of gas exchange $P_{GE}$.

In some applications, the input gas mixture and/or the output gas mixture may have a very high moisture content. For example, when using the gas analyser 105 for determining a measure of gas exchange over an oxygenator or the lungs of a mechanically ventilated patient, the water vapour content in the gas outlet line 103 is typically very high. In such scenarios, in order to avoid condensation of water vapour in the gas analyser 105, the second sampling line 107 may comprise a water vapour trap or gas sample conditioner (not shown) for conditioning and especially for drying the gas samples withdrawn from the gas outlet line 103 before the gas samples enter the gas analyser 105. The gas sample conditioner may, e.g., comprise a piece of Nafion tubing or silica gel. Due to the removal of water by the gas sample conditioner, the composition of the gas samples measured upon is not the same as the composition of the output gas mixture in the gas outlet line 103. Therefore, the fraction of the specific gas, measured by the sidestream gas analyser 105 will not accurately reflect the fraction of the specific gas in the gas outlet line 103.

To this end, in situations where the system 100 comprises a gas sample conditioner for removal of water vapour in the gas samples withdrawn from the gas outlet line 103 and/or the gas inlet line 101, prior to analysis of the gas samples by the gas analyser 105, the at least one processor 37 may be configured to determine the output fraction of the specific gas in the output gas mixture based on a fraction of the specific gas in an analysed output gas sample and an estimated removal of water vapour ($\Delta FH2O_{out}$) from the output gas sample, and/or to determine the input fraction of the specific gas in the input gas mixture based on a fraction of the specific gas in an analysed input gas sample and an estimated removal of water vapour ($\Delta FH2O_{in}$) from the input gas sample by the gas sample conditioner. How to determine $\Delta FH2O_{out}$ and/or $\Delta FH2O_{in}$ will be discussed in more detail below.

The gas analyser 105 will now be described in the context of a combined system for extracorporeal blood gas exchange via a membrane oxygenator during lung protective ventilation of the patient using a mechanical ventilator.

Fig. 2 illustrates a system 1 for combined mechanical ventilation of the lungs of a patient 3 and extracorporeal removal of $CO_2$ from the blood of the patient 3. The system 1 will hereinafter be referred to as an ECMO-vent system. ECMO (extracorporeal membrane oxygenation) is one of several terms used for extracorporeal blood gas exchange where blood is pumped outside the body of a treated patient to a device, sometimes referred to as a heart-lung machine, which removes $CO_2$ and sends oxygen-enriched blood back to the patient. Other terms that are frequently used in the art for the same or similar treatments are ECLA (extracorporeal lung assist), ECCO2R (extracorporeal $CO_2$ removal), ECLS (extracorporeal life support), and ECGE (extracorporeal membrane gas-exchange), all of which are encompassed by the term ECMO as used herein.

The ECMO-vent system 1 comprises a device 5, hereinafter referred to as an ECMO device, for extracorporeal removal of $CO_2$ from the blood of the patient 3, and a mechanical ventilator 7 for mechanically ventilating the patient 3 through the supply of breathing gas to the lungs of the patient.

The ventilator 7 comprises or is connected to a source of pressurised breathing gas (not shown), which breathing gas is supplied to the patient 3 via a patient circuit 9. In this example, the patient circuit 9 comprises an inspiratory line 11 for conveying a flow of breathing gas to the patient 3, and an expiratory line 13 for conveying a flow of exhalation gas exhaled by the patient away from the patient. The inspiratory line 11 and the expiratory line 13 are connected to each other via a so called Y-piece 15 which, in turn, is connected to the patient 3 via a common line 17.

The ECMO device 5 is configured to provide ECMO treatment to the patient 3 by generating an extracorporeal flow of blood from the patient 3, oxygenating the blood through extracorporeal blood gas exchange in which $CO_2$ is removed from, and oxygen added to, the extracorporeal blood flow, and returning the oxygen-enriched blood to the patient 3.

To generate the flow of blood to and from the patient 3, the ECMO device 5 may comprise a blood flow generator (not shown), typically in form of one or several roller, turbine and/or centrifugal pumps. The blood flow generator generates a flow of blood through a tubing system forming a blood flow channel 19 of the ECMO device 5, where parts of the channel may be heated to maintain a desired temperature of the blood when returned to the patient 3.

The blood gas exchange, including blood oxygenation and $CO_2$ removal, takes place in a membrane oxygenator 21 of the ECMO device 5, in which an oxygen-containing sweep gas flow interacts with the blood in the blood flow channel 19 via a membrane 23 of the oxygenator 21. The membrane 23 acts as a gas-liquid barrier enabling transfer of $CO_2$ and $O_2$ content between the bloodstream flowing through the oxygenator 21 on a liquid-side of the membrane 23 and the sweep gas flow flowing through the oxygenator 21 on a gas-side of the membrane 23.

The sweep gas flow is generated by a sweep gas generator 25 connected to one or more sweep gas sources, typically including one or both of an oxygen source and a source of compressed air. In this exemplary embodiment, the sweep gas generator 25 is further connected to a CO2 source in order to control the degree of CO2 removal over the oxygenator 21 through addition of CO2 to the sweep gas flow. The sweep gas generator 23 is configured to deliver a controllable sweep gas composition to the oxygenator 21 at a controllable sweep gas flow rate.

The composition and, optionally, the flow rate of the sweep gas generated by the sweep gas generator 23, may be automatically controlled by a controller or control computer 27 of the ECMO device 5 based on set target values and sensor data obtained by various sensors 29, 31 of the ECMO device 5. In particular, the control computer 27 of the ECMO device 5 may be configured to automatically control an addition of CO2 to a sweep gas flow comprising any or both of oxygen and air, based on a set target for a measure of CO2 removal by the oxygenator 21.

Hereinafter, the sweep gas flow upstream of the oxygenator 21 (i.e., before the oxygenator from the sweep gas' point of view) will be referred to as an input sweep gas flow or a pre-oxygenator sweep gas flow, and the sweep gas flow downstream of the oxygenator 21 (i.e., after the oxygenator from the sweep gas' point of view) will be referred to as an output sweep gas flow or a post-oxygenator sweep gas flow. The input sweep gas flow flows from the sweep gas generator 25 to the oxygenator 21 via a sweep gas inlet line 33a of the ECMO device 5, and the output sweep gas flow flows from the oxygenator 21 to atmosphere or an evacuation or recirculation system via a sweep gas outlet line 33b. In most configurations, ECMO systems are open systems, meaning that the post oxygenator sweep gas flow is allowed to escape into the ambient. In some cases, especially when anesthetic agents are added to the sweep gas flow, a closed or semi closed (sweep) gas control system can be envisioned, similar to gas control systems often used in anesthesia machines.

Likewise, the bloodstream upstream of the oxygenator 21 (i.e., before the oxygenator from the bloodstream's point of view) may hereinafter be referred to as an input bloodstream or pre-oxygenator bloodstream, and the bloodstream downstream of the oxygenator 21 (i.e., after the oxygenator from the bloodstream's point of view) may be referred to as an output bloodstream or post-oxygenator bloodstream. The input bloodstream flows from the patient 3 to the oxygenator 21 via a bloodstream inlet line 19a of the ECMO device 5, and the output bloodstream flows from the oxygenator 21 and back to the patient 3 via a bloodstream outlet line 19b of the ECMO device 5.

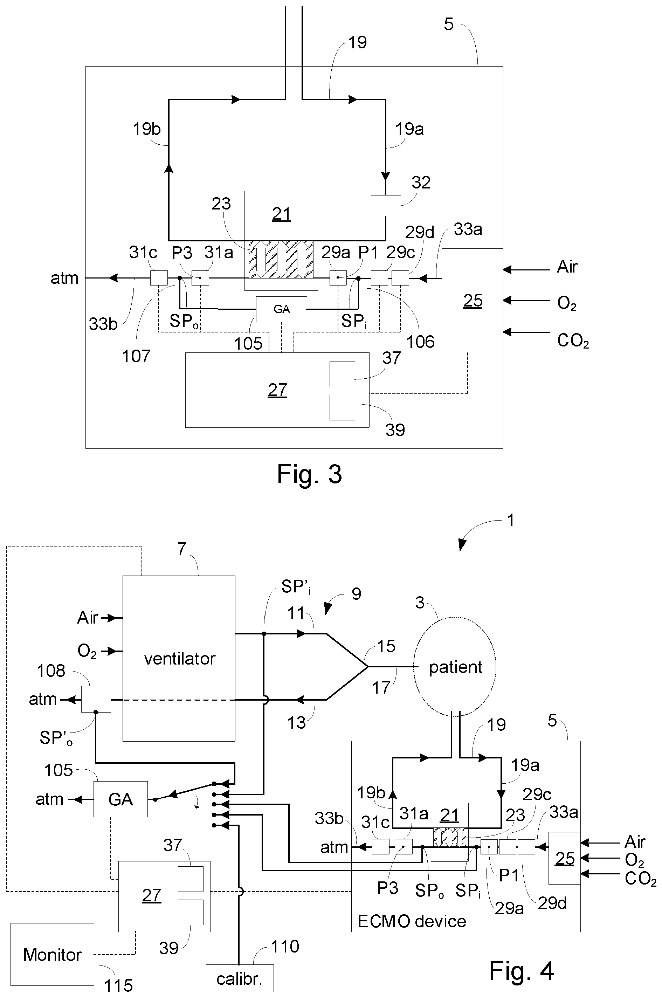

With reference now made to Fig. 3, illustrating a first exemplary sensor configuration of the ECMO device 5, the sensors 29, 31 of the ECMO device 5 may comprise:

- a pre-oxygenator flow rate sensor 29a for measuring a flow rate of the input sweep gas flow, $\dot{V}_{in}$. The pre-oxygenator flow rate sensor 29a is a mainstream flow sensor, meaning that it is configured to measure the flow rate of the sweep gas flowing in the sweep gas inlet line 33a. The pre-oxygenator flow measurements obtained by the pre-oxygenator flow rate sensor 29a take place at a pre-oxygenator point of flow measurement denoted P1 in the sweep gas inlet line 33a.
- a pre-oxygenator temperature sensor 29c for measuring a temperature of the input sweep gas, $T_{in,gas}$.
- a pressure sensor 29d for measuring a sweep gas circuit pressure, $P_{gas}$, substantially corresponding to the sweep gas pressure in the gas inlet line 33a.
- a post-oxygenator flow rate sensor 31a for measuring a flow rate of the output sweep gas flow, $\dot{V}_{out}$. The post-oxygenator flow rate sensor 31a is a mainstream flow sensor, meaning that it is configured to measure the flow rate of the sweep gas flowing in the sweep gas outlet line 33b. The post-oxygenator flow measurements obtained by the post-oxygenator flow rate sensor 31a take place at a post-oxygenator point of flow measurement denoted P3 in the sweep gas outlet line 33b.
- a post-oxygenator temperature sensor 31c for measuring a temperature of the output sweep gas.
- a multisampling sidestream gas analyser 105 as illustrated and discussed with reference to Fig. 1.

The first sampling line 106 of the multisampling sidestream gas analyser 105 is connected to the sweep gas inlet line 33a of the ECMO device 5 and the second sampling line 107 of the gas analyser 105 is connected to the sweep gas outlet line 33b. The gas analyser 105 is configured to measure at least a fraction of CO2 ($FCO2_{in}$) in samples of the input sweep gas flow and a fraction of CO2 ($FCO2_{out}$) in samples of the output sweep gas flow. The gas analyser 105 may also be configured to measure a fraction of one or more additional gases selected from the group consisting of oxygen (O2), nitrogen gas (N2) and anaesthetic agents in the samples of the inlet sweep gas flow and the outlet sweep gas flow. Notably, the gas analyser 105 is configured to measure the fraction of CO2 and, optionally, the fraction of the at least one additional gas in the sweep gas samples at a point of gas analysis that is separated in distance from the point of pre-oxygenator sweep gas flow rate measurements, P1, at least by the length of the first sampling line 106, and from the point of post-oxygenator sweep gas flow rate measurements, P3, at least be the length of the second sampling line 107. In this embodiment, the gas analyser comprises at least a CO2 sensor and an O2 sensor for measuring a fraction of CO2 and O2, respectively, in the sweep gas samples.

In some embodiments, the ECMO device 5 may further comprise or be connected to a pre-oxygenator blood gas analyser 32 for measuring a partial pressure of at least CO2 in the input bloodstream, $PCO2_{in}$. The pre-oxygenator blood gas analyser 32 may also be configured to measure a partial pressure of O2 in the input bloodstream, $PO2_{in}$. The pre-

oxygenator blood gas analyser 32 may also be configured to measure a haemoglobin content of the input bloodstream, $Hb_{in}$. In some embodiments, the blood gas analyser 32 is not incorporated into the ECMO device 5 but arranged to form part of another medical device that is connected to the ECMO device 5 in order for the ECMO device 5 to receive measurements obtained by the blood gas analyser. For example, the blood gas analyser may form part of a stand-alone blood gas analyser unit, often referred to as a BGA, commonly used for intermittent blood gas analysis during ECMO treatments.

Fig. 4 illustrates another exemplary embodiment of the ECMO-vent system 1, wherein the gas analyser 105 is connected to both the sweep gas inlet line 33a and the sweep gas outline line 33b of the ECMO device 5, and to the inspiratory line 11 and the expiratory line 13 of the ventilator 7.

The gas analyser 105 is configured to sequentially receive and analyse gas samples from the sweep gas inlet line 33a and the sweep gas outlet line 33b of the ECMO device 5 in order to analyse a presence of a specific gas in the input sweep gas flow and in the output sweep gas flow, and to sequentially receive and analyse gas samples from the inspiratory line 11 and the expiratory line 13 of the mechanical ventilator 7, in order to analyse a presence of the specific gas in the flow of breathing gas and in the flow of exhalation gas.

The at least one processor 37 of the control computer 27 may then be configured to determine a measure of gas exchange of the specific gas in the oxygenator 21 based on the analysis of the gas samples from the sweep gas inlet line 33a and the sweep gas outlet line 33b, and to determine a measure of gas exchange of the specific gas in the lungs of the patient 3 based on the analysis of the gas samples from the inspiratory line 11 and the expiratory line 13.

The gas analyser 105 may be connected to any part of the inspiratory line 11 and the expiratory line 13 for withdrawal of breathing gas samples and exhalation gas samples, respectively. In the illustrated embodiment, the gas analyser 105 is connected to a mixing chamber 108 that forms part of the expiratory line 13, which mixing chamber serves to collect and mix exhalation gases exhaled by the patient 3 during the course of an expiration phase.

As illustrated in Fig. 4, the gas analyser 105 may further be connected to a source of calibration gas 110 and configured to sequentially receive and analyse gas samples also from the calibration gas source 110, in order for the gas analyser 105 to be periodically or intermittently calibrated, or in order to periodically or intermittently verify the accuracy of the gas analyser 105.

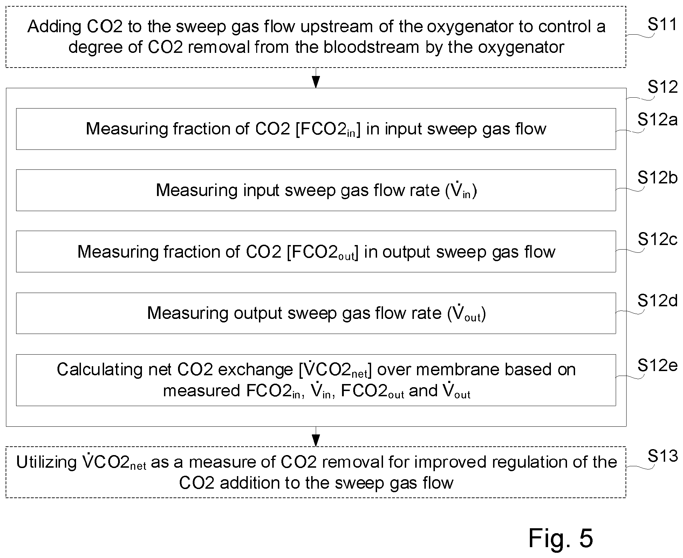

With simultaneous reference to previous drawings, some functions and features of the ECMO-vent system 1 will now be described with reference to the flowchart shown in Fig. 5, which flowchart illustrate a method for controlling a degree of CO2 removal in and by the ECMO device 5. The method is a computer-implemented method that is performed by the ECMO device 5 upon execution of a computer program by at least one processor 37 of the control computer 27. The computer program(s) comprise computer-readable instructions that may be stored in a storage medium of the ECMO-vent system 1, such as a non-transitory hardware memory device 39 of the control computer 27.

In a first optional step, S11, CO2 is added to the sweep gas flow upstream of the oxygenator 21 to control a degree of CO2 removal from the bloodstream of the patient 3 by the oxygenator 21.

In a second step, S12, a net CO2 exchange ($\dot{V}CO2_{net}$) over the membrane 23 of the oxygenator 21 is calculated.

Step S12 comprises the following sub-steps:

S12a)  measuring, with the gas analyser 105, a pre-oxygenator fraction of CO2 ($FCO2_{in}$) in the sweep gas flow upstream of the oxygenator 21;

S12b)  measuring, with the pre-oxygenator flow rate sensor 29a, a pre-oxygenator sweep gas flow rate ($\dot{V}_{in}$) of the sweep gas flow upstream of the oxygenator 21;

S12c)  measuring, with the gas analyser 105, a post-oxygenator fraction of CO2 ($FCO2_{out}$) in the sweep gas flow downstream of the oxygenator 21;

S12d)  measuring, with the post-oxygenator flow rate sensor 31a, a post-oxygenator sweep gas flow rate ($\dot{V}_{out}$) of the sweep gas flow downstream of the oxygenator 21, and

S12e)  calculating $\dot{V}CO2_{net}$ over the membrane 23 based on measured $FCO2_{in}$, $\dot{V}_{in}$, $FCO2_{out}$ and $\dot{V}_{out}$.

In a third optional step, S13, $\dot{V}CO2_{net}$ is utilized as a measure of CO2 removal for improved regulation of the CO2 addition to the sweep gas flow. Step S13 may comprise any of, or any combination of:

- presenting $\dot{V}CO2_{net}$ to an operator of the ECMO device 5 as decision support in manual adjustment of the addition of CO2 to the sweep gas flow, and/or

- presenting, to the operator, a recommendation for adjustment of the addition of CO2 to the sweep gas flow, based on $\dot{V}CO2_{net}$ and a set target for CO2 removal by the oxygenator, and/or

- automatically regulating the addition of CO2 to the sweep gas flow based on $\dot{V}CO2_{net}$.

$\dot{V}CO2_{net}$ may be calculated as the fraction of CO2 ($FCO2_{in}$) in the input sweep gas flow times the input sweep gas

flow rate ($\dot{V}_{in}$), minus the fraction of CO2 ($FCO2_{out}$) in the output sweep gas flow times the output sweep gas flow rate ($\dot{V}_{out}$), in accordance with:

$$VCO2_{net} = (FCO2_{in} * \dot{V}_{in}) - (FCO2_{out} * \dot{V}_{out}) \qquad \text{eq. 1}$$

Of course, in order to accurately calculate the flow of CO2 going into and out from the oxygenator 21, the terms $FCO2_{in}$ and $FCO2_{out}$ should reflect the true fractions of CO2 at the points of measurements of $\dot{V}_{in}$ and $\dot{V}_{out}$. Due to the humid environment (often close to 100% RH) downstream of the oxygenator 21, there is often a need for conditioning (e.g., drying) the sweep gas before it enters the gas analyser, e.g., to prevent condensation of water vapour inside the gas analyser 105. Therefore, with reference again made to Fig. 3, the ECMO device 5 may comprise a water vapour trap or gas sample conditioner (not shown) for conditioning and especially for drying the sweep gas samples withdrawn from the sweep gas outlet line 33b before the sweep gas samples enter the gas analyser 105. The gas sample conditioner may, e.g., comprise a piece of Nafion tubing or silica gel. In some embodiments, the gas sample conditioner is arranged to form part of, or to be arranged within, the second sampling line 107 of the gas analyser 105. Due to the removal of water by the gas sample conditioner, the composition of the post-oxygenator sweep gas samples measured upon is not the same as the composition of the sweep gas flow in the sweep gas outlet line 33b, where $\dot{V}_{out}$ is measured. Therefore, the fraction of CO2, $FCO2_{out}$, measured by the sidestream gas analyser 105 at the point of gas analysis will not accurately reflect the fraction of CO2 at the point of output sweep gas flow rate measurements P3.

To this end, the method may further comprise the steps of:

- calculating a compensated pre-oxygenator fraction of CO2 ($FCO2_{in,comp}$) representing an estimate of a fraction of CO2 at the point of measurement, P1, of $\dot{V}_{in}$, based on $FCO2_{in}$ and an estimated addition or removal of water vapour, $\Delta FH2O_{in}$, to or from the sweep gas between the point of measurement, P1, of $\dot{V}_{in}$ and a point of measurement of $FCO2_{in}$, and/or
- calculating a compensated post-oxygenator fraction of CO2 ($FCO2_{out,comp}$) representing an estimate of a fraction of CO2 at a point of measurement, P3, of $\dot{V}_{out}$, based on $FCO2_{out}$ and an estimated addition or removal of water vapour ($\Delta FH2O_{out}$) to or from the sweep gas between the point of measurement, P3, of $\dot{V}_{out}$ and a point of measurement of $FCO2_{out}$, and
- calculating $\dot{V}CO2_{net}$ based on $\dot{V}_{in}$ and $\dot{V}_{out}$, and at least one of $FCO2_{in,comp}$ and $FCO2_{out,comp}$.

For example, a compensated post-oxygenator fraction of CO2 ($FCO2_{out,comp}$) can be calculated as a function of measured post-oxygenator fraction of CO2 ($FCO2_{out}$) and an estimated addition or removal of water vapour ($\Delta FH2O_{out}$) to or from the sweep gas between the point of measurement, P3, of $\dot{V}_{out}$ and the point of measurement of $FCO2_{out}$ (i.e., the point of gas analysis, corresponding to the location of the gas analyser 105), and used in the determination of $\dot{V}CO2_{net}$ according to:

$$FCO2_{out,comp} = f(FCO2_{out}, \Delta FH2O_{out}) \qquad \text{eq. 2}$$

$$\dot{V}CO2_{net} = (FCO2_{in} * \dot{V}_{in}) - (FCO2_{out,comp} * \dot{V}_{out}) \qquad \text{eq. 3}$$

The estimated addition or removal of water vapour, $\Delta FH2O_{out}$, may, in some embodiments, be calculated based on: a measured post-oxygenator temperature ($T_{out,gas}$) of the sweep gas flow downstream of the oxygenator 21, measured by the post-oxygenator temperature sensor 31c; a measured or estimated post-oxygenator relative humidity ($RH_{out}$) of the sweep gas flow downstream of the oxygenator 21; a measured or estimated reference temperature ($T_{ref}$) at or close to the point of measurement of $FCO2_{out}$, and a measured or estimated reference relative humidity ($RH_{ref}$) at or close to the point of measurement of $FCO2_{out}$. In embodiments where the gas sample conditioner comprises a piece of Nafion tubing, the reference relative humidity, $RH_{ref}$, can be assumed to correspond to the relative humidity of the air surrounding the Nafion tubing. In embodiments where the gas sample conditioner comprises silica gel, the reference relative humidity, $RH_{ref}$, can be assumed to be zero or near zero. The post-oxygenator relative humidity, $RH_{out}$ can in most situations be assumed to be 100% but may, in some embodiments, be measured by a humidity sensor (not shown) of the ECMO device 5, arranged downstream of the oxygenator 21.

Another potential source of error in the calculation of $\dot{V}CO2_{net}$ is inaccuracy in sweep gas flow rate measurements. The flow sensors 29a and 31a for measuring $\dot{V}_{in}$ and $\dot{V}_{out}$ are normally calibrated for a specific gas composition and deviations between an assumed composition and an actual composition of the sweep gas measured upon introduces errors in flow rate determinations. Therefore, the method may further comprise the steps of:

- measuring or estimating a pre-oxygenator fraction of at least one additional gas in the sweep gas flow upstream of the oxygenator 21, the at least one additional gas being one or more of water vapour (H2O), O2, nitrogen gas (N2), and an anaesthetic agent, and/or

- measuring or estimating a post-oxygenator fraction of the at least one additional gas in the sweep gas flow downstream of the oxygenator, and
- calculating a compensated pre-oxygenator sweep flow rate ($\dot{V}_{in,comp}$) based on $\dot{V}_{in}$, $FCO2_{in}$ and the pre-oxygenator fraction of the at least one additional gas, and/or
- calculating a compensated post-oxygenator sweep flow rate ($\dot{V}_{out,comp}$) based on $\dot{V}_{out}$, $FCO2_{out}$ and the post-oxygenator fraction of the at least one additional gas, and
- calculating $\dot{V}CO2_{net}$ based on $FCO2_{in}$, $FCO2_{out}$ and at least one of $\dot{V}_{in,comp}$ and $\dot{V}_{out,comp}$.

By taking the composition of the sweep gas into account and compensating the pre-oxygenator and/or the post-oxygenator sweep flow rate measurements $\dot{V}_{in}$ and $\dot{V}_{out}$ based on a measured fraction of at least one additional gas in the sweep gas flow upstream and/or downstream the oxygenator, a more accurate value of $\dot{V}CO2_{net}$ can be obtained.

For example, a compensated post-oxygenator sweep gas flow rate, $\dot{V}_{out,comp}$, may be calculated as a function of $\dot{V}_{out}$, $FCO2_{out}$ and a post-oxygenator fraction of O2, water vapour and/or N2, according to:

$$\dot{V}_{out,comp} = f\ (\dot{V}_{out},\ FCO2_{out},\ FO2_{out},\ FH2O_{out},\ FN2_{out}), \qquad \text{eq. 4}$$

where $FCO2_{out}$ and $FO2_{out}$ are the fractions of CO2 and O2 measured by the sidestream post-oxygenator gas analyser 31b, $FH2O_{out}$ is the fraction of water vapour in the sweep gas flow downstream of the oxygenator, which may be determined from the measured post-oxygenator temperature, $T_{out,gas}$, of the sweep gas flow downstream of the oxygenator and the measured or estimated post-oxygenator relative humidity, $RH_{out}$, of the sweep gas flow downstream of the oxygenator, and $FN2_{out}$ is the post-oxygenator fraction of N2 which may be measured by the post-oxygenator gas analyser 31b or be assumed to correspond to the remaining fraction of the post-oxygenator sweep gas flow.

$\dot{V}CO2_{net}$ may then be calculated as:

$$\dot{V}CO2_{net} = (FCO2_{in} * \dot{V}_{in}) - (FCO2_{out,comp} * \dot{V}_{out,comp}) \qquad \text{eq. 5}$$

As discussed above, there is often a need for using a gas sample conditioner between the sweep gas sampling point in the sweep gas outlet line 33b and the sidestream gas analyser 105. The removal or reduction of water vapour content by the gas sample conditioner introduces a deviation between the composition of the sweep gas in the sweep gas outlet line (where $\dot{V}_{out}$ is measured) and the sweep gas samples measured upon by the sidestream gas analyser 105. Therefore, to further improve accuracy in the determination of the compensated post-oxygenator sweep gas flow rate, $\dot{V}_{out,comp}$, the compensated post-oxygenator fraction of CO2, $FCO2_{out,comp}$, may be advantageously used instead of the measured post-oxygenator fraction of CO2, $FCO2_{out}$, in the determination of $\dot{V}_{out,comp}$, in accordance with:

$$\dot{V}_{out,comp} = f\ (\dot{V}_{out},\ FCO2_{out,comp},\ FO2_{out},\ FH2O_{out},\ FN2_{out}) \qquad \text{eq. 6}$$

Any fractions of additional gases measured by the post-oxygenator sidestream gas analyser 31b, such as measured fractions of O2 ($FO2_{out}$) and/or measured fractions of N2 ($FN2_{out}$), may also be compensated based on the estimated addition or removal of water vapour, $\Delta FH2O_{out}$, to or from the sweep gas between the point of measurement, P3, of $\dot{V}_{out}$ and the point of measurement, P4, of the additional gas by the gas analyser. A compensated fraction of the additional gas may be calculated as a function of the measured fraction of the additional gas and the estimated addition or removal of water vapour, $\Delta FH2O_{out}$, in accordance with:

$$FO2_{out,comp} = f\ (FO2_{out},\ \Delta FH2O_{out}) \qquad \text{eq. 7}$$

$$FN2_{out,comp} = f\ (FN2_{out},\ \Delta FH2O_{out}) \qquad \text{eq. 8}$$

The compensated fractions of the additional gases may then replace the measured fractions of additional gases in eq. 6 to further improve the accuracy in post-oxygenator sweep gas flow rate determination, in accordance with:

$$\dot{V}_{out,comp} = f\ (\dot{V}_{out},\ FCO2_{out,comp},\ FO2_{out,comp},\ FH2O_{out,comp},\ FN2_{out,comp}) \qquad \text{eq. 9}$$

A precise measure of $\dot{V}CO2_{net}$ may then be calculated using eq. 5 with a $\dot{V}_{out,comp}$ value calculated in accordance with eq. 9.

It should be noted that although described herein as a technique for controlling *removal* of CO2 from the bloodstream of a patient, the technique could as well be used to control an *addition* of CO2 to the bloodstream of the patient via the oxygenator 21. This may sometimes be desired to obtain or maintain a desired pH or partial pressure of CO2 (PCO2) in the blood of the patient 3. Consequently, it should be realised that the disclosed method for controlling CO2 removal in the ECMO device 5 could be generalised to methods for controlling transfer of CO2 to or from the bloodstream of the patient 3, via the oxygenator 21, in order to control addition or removal of CO2 to or from the blood of the patient. The purpose of controlling CO2 transfer may be any of: 1) ensuring sufficient removal of CO2 from the blood of the patient; 2) keeping CO2 removal constant, e.g., in order to evaluate an ongoing respiratory treatment provided by the mechanical ventilator 7 and/or a lung function of the patient 3, and 3) obtaining or maintaining a set or desired post-oxygenator pH and/or PCO2, in particular during adjustments of the sweep gas flow rate.

In applications where CO2 transfer over the oxygenator membrane 23 is controlled to obtain or maintain a set or desired post-oxygenator pH and/or PCO2, the control computer 27 of the ECMO device 5 may be configured to automatically control the inlet sweep gas flow rate and/or the addition of CO2 to the inlet sweep gas flow to obtain or maintain pH and/or PCO2.

## Claims

1. A system (1) for analysing gas concentrations before and after a point of gas exchange (3, 23), the system (1) comprising:

   a device (5) for extracorporeal blood gas exchange, the device (5) comprising:

   - an oxygenator (21) including a membrane (23) acting as a gas-liquid barrier enabling CO2 transfer between a bloodstream and a sweep gas flow through the oxygenator, wherein the membrane (23) constitutes a first point of gas exchange, and
   - a sweep gas inlet line (33a) for conveying an input sweep gas towards the membrane (23), and a sweep gas outlet line (33b) for conveying an output sweep gas away from the membrane (23), and

   a mechanical ventilator (7) for mechanically ventilating a patient (3) through the supply of breathing gas to the lungs of the patient (3), wherein the lungs of the patient (3) constitutes a second point of gas exchange, the ventilator (7) comprising:

   - an inspiratory line (11) for conveying a flow of breathing gas towards the patient (3), and an expiratory line (13) for conveying a flow of exhalation gas away from the patient (3),

   **characterised in that** a gas analyser (105) is connected to both the sweep gas inlet line (33a), the sweep gas outline line (33b), the inspiratory line (11), and the expiratory line (13) and configured to sequentially receive and analyse gas samples from the sweep gas inlet line (33a), the sweep gas outlet line (33b), the inspiratory line (11), and the expiratory line (13), the gas analyser comprising at least one processor (37) configured to determine a measure of gas exchange of the specific gas at the first point of gas exchange based on the analysis of the gas samples from the sweep gas inlet line (33a) and the sweep gas outlet line (33b), and to determine a measure of gas exchange of the specific gas at the second point of gas exchange based on the analysis of the gas samples from the inspiratory line (11) and the expiratory line (13).

2. The system (100; 1) of claim 1, wherein the at least one processor (37) is configured to:

   - determine an input fraction of the specific gas in the input sweep gas based on the analysis by the gas analyser (105) of the gas samples from the sweep gas inlet line (33a),
   - determine an output fraction of the specific gas in the output sweep gas based on the analysis by the gas analyser (105) of the gas samples from the sweep gas outlet line (33b), and
   - determine, based on the input and output fractions of the specific gas, a measure of gas exchange of the specific gas at the first point of gas exchange (23).

3. The system (1) of claim 2, wherein the at least one processor (37) is configured to:

   - receive a measurement of a flow rate $[\dot{V}_{in}]$ of the input sweep gas in the sweep gas inlet line (33a),
   - receive a measurement of a flow rate $[\dot{V}_{out}]$ of the output sweep gas in the sweep gas outlet line (33b),

- determine, based on the input fraction of the specific gas in the input sweep gas and the flow rate of the input sweep gas, an input net flow of the specific gas in the sweep gas inlet line (33a),
- determine, based on the output fraction of the specific gas in the output sweep gas and the flow rate of the output sweep gas, a net flow of the specific gas in the sweep gas outlet line (33b), and
- determine a change in net flow of the specific gas at the first point of gas exchange (23) based on the input net flow and the output net flow of the specific gas, which change in net flow is indicative of a gas exchange of the specific gas at the first point of gas exchange (3).

4. The system (1) of any of the preceding claims, wherein the at least one processor (37) is configured to:

- determine an input fraction of the specific gas in the breathing gas based on the analysis by the gas analyser (105) of the gas samples from the inspiratory line (11),
- determine an output fraction of the specific gas in the exhalation gas based on the analysis by the gas analyser (105) of the gas samples from the expiratory line (13), and
- determine, based on the input and output fractions of the specific gas, a measure of gas exchange of the specific gas at the second point of gas exchange (3).

5. The system (1) of claim 4, wherein the at least one processor (37) is configured to:

- receive a measurement of a flow rate $[\dot{V}_{in}]$ of the breathing gas in the inspiratory line (11),
- receive a measurement of a flow rate $[\dot{V}_{out}]$ of the exhalation gas in the expiratory line (13),
- determine, based on the input fraction of the specific gas in the breathing gas and the flow rate of the breathing gas, an input net flow of the specific gas in the inspiratory line (11),
- determine, based on the output fraction of the specific gas in the exhalation gas and the flow rate of the exhalation gas, a net flow of the specific gas in the expiratory line (13), and
- determine a change in net flow of the specific gas at the first point of gas exchange (3) based on the input net flow and the output net flow of the specific gas, which change in net flow is indicative of a gas exchange of the specific gas at the second point of gas exchange (3).

6. The system (1) of any of the preceding claims, wherein the system (1) comprises a gas sample conditioner for removal of water vapour in the gas samples from the sweep gas inlet line (33a) and/or the gas samples from the sweep gas outlet line (33b), prior to analysis of the gas samples by the gas analyser (105), the at least one processor (37) being configured to:

- determine the input fraction of the specific gas in the input sweep gas based on a fraction of the specific gas in an analysed input sweep gas sample and an estimated removal of water vapour $[\Delta FH2O_{in}]$ from the input sweep gas sample by the gas sample conditioner, and/or
- determine the output fraction of the specific gas in the output sweep gas based on a fraction of the specific gas in an analysed output sweep gas sample and an estimated removal of water vapour $[\Delta FH2O_{out}]$ from the output sweep gas sample by the gas sample conditioner.

7. The system (1) of any of the preceding claims, wherein the system (1) comprises a gas sample conditioner for removal of water vapour in the gas samples from the inspiratory line (11) and/or the gas samples from the expiratory line (13), prior to analysis of the gas samples by the gas analyser (105), the at least one processor (37) being configured to:

- determine the input fraction of the specific gas in the breathing gas based on a fraction of the specific gas in an analysed breathing gas sample and an estimated removal of water vapour $[\Delta FH2O_{in}]$ from the breathing gas sample by the gas sample conditioner, and/or
- determine the output fraction of the specific gas in the exhalation gas based on a fraction of the specific gas in an analysed exhalation gas sample and an estimated removal of water vapour $[\Delta FH2O_{out}]$ from the exhalation gas sample by the gas sample conditioner.

8. The system (1) of any of the preceding claims, wherein the gas analyser (105) is connected also to a calibration gas source (110) for delivery of a calibration gas, the gas analyser (105) being configured to sequentially receive and analyse gas samples from the sweep gas inlet line (33a), the sweep gas outlet line (33b), the inspiratory line (11), the expiratory line (13), and the calibration gas source (110).

9. The system (1) of any of the preceding claims, wherein the specific gas is selected from the group consisting of carbon dioxide [CO2] and oxygen [O2].

10. The system (1) of any of the preceding claims, wherein the gas analyser (105) comprises a first sensor for measuring a fraction of CO2 [$FCO2_{in}$] in the input sweep gas and the inspiration gas and a fraction of CO2 [$FCO2_{out}$] in the output sweep gas and the exhalation gas, and a second sensor for measuring a fraction of O2 [$FO2_{in}$] in the input sweep gas and the inspiration gas and a fraction of O2 [$FO2_{out}$] in the output sweep gas and the exhalation gas.

11. The system (1) of claim 10, wherein the first sensor is a non-dispersive infrared [NDIR] CO2 sensor and the second sensor is a paramagnetic or electrochemical O2 sensor.

12. The system (1) of any of the preceding claims, wherein the system (1) is configured to display gas exchange information on a monitor (115) of the system (1), the gas exchange information comprising information related to the measure of gas exchange of the specific gas at the first point of gas exchange (23), information related to the measure of gas exchange of the specific gas at the second point of gas exchange (3), and/or information related to a measure of total gas exchange of the specific gas at the first and the second points of gas exchange (3, 23).

**Patentansprüche**

1. System (1) zum Analysieren von Gaskonzentrationen vor und nach einem Gasaustauschpunkt (3,23), wobei das System (1) Folgendes umfasst:

   eine Vorrichtung (5) zum extrakorporalen Blutgasaustausch, wobei die Vorrichtung (5) Folgendes umfasst:

   - einen Sauerstoffgenerator (21), der eine Membran (23) beinhaltet, die als eine Gas-Flüssigkeit-Barriere fungiert, die CO2-Austausch zwischen einem Blutstrom und einer Spülgasströmung durch den Sauerstoffgenerator ermöglicht, wobei die Membran (23) einen ersten Gasaustauschpunkt bildet, und
   - eine Spülgaseinlassleitung (33a) zum Transportieren eines Eingabespülgases in Richtung der Membran (23) und eine Spülgasauslassleitung (33b) zum Transportieren eines Ausgabespülgases weg von der Membran (23) und

   ein mechanisches Beatmungsgerät (7) zum mechanischen Beatmen eines Patienten (3) durch die Zufuhr von Atemgas in die Lungen des Patienten (3), wobei die Lungen des Patienten (3) einen zweiten Gasaustauschpunkt bilden und das Beatmungsgerät (7) Folgendes umfasst:

   - eine Inspirationsleitung (11) zum Transportieren einer Atemgasströmung in Richtung des Patienten (3) und eine Exspirationsleitung (13) zum Transportieren einer Ausatemgasströmung weg von dem Patienten (3),

   **dadurch gekennzeichnet, dass** ein Gasanalysator (105) jeweils mit der Spülgaseinlassleitung (33a), der Spülgasumrissleitung (33b), der Inspirationsleitung (11) und der Exspirationsleitung (13) verbunden und dazu konfiguriert ist, aufeinanderfolgend Gasproben von der Spülgaseinlassleitung (33a), der Spülgasauslassleitung (33b), der Inspirationsleitung (11) und der Exspirationsleitung (13) aufzunehmen und zu analysieren, wobei der Gasanalysator mindestens einen Prozessor (37) umfasst, der dazu konfiguriert ist, ein Maß an Gasaustausch des spezifischen Gases an dem ersten Gasaustauschpunkt basierend auf der Analyse der Gasproben aus der Spülgaseinlassleitung (33a) und der Spülgasauslassleitung (33b) zu bestimmen und ein Maß an Gasaustausch des spezifischen Gases an dem zweiten Gasaustauschpunkt basierend auf der Analyse der Gasproben aus der Inspirationsleitung (11) und der Exspirationsleitung (13) zu bestimmen.

2. System (100; 1) nach Anspruch 1, wobei der mindestens eine Prozessor (37) zu Folgendem konfiguriert ist:

   - Bestimmen eines Eingabeanteils des spezifischen Gases in dem Eingabespülgas basierend auf der Analyse durch den Gasanalysator (105) der Gasproben aus der Spülgaseinlassleitung (33a),
   - Bestimmen eines Ausgabeanteils des spezifischen Gases in dem Ausgabespülgas basierend auf der Analyse durch den Gasanalysator (105) der Gasproben aus der Spülgasauslassleitung (33b) und
   - Bestimmen, basierend auf dem Eingabe- und dem Ausgabeanteil des spezifischen Gases, eines Maßes an Gasaustausch des spezifischen Gases an dem ersten Gasaustauschpunkt (23).

3. System (1) nach Anspruch 2, wobei der mindestens eine Prozessor (37) zu Folgendem konfiguriert ist:

- Empfangen einer Messung einer Strömungsrate [$V_{in}$] des Eingabespülgases in der Spülgaseinlassleitung (33a),
- Empfangen einer Messung einer Strömungsrate [$V_{out}$] des Ausgabespülgases in der Spülgasauslassleitung (33b),
- Bestimmen, basierend auf dem Eingabeanteil des spezifischen Gases in dem Eingabespülgas und der Strömungsrate des Eingabespülgases, einer Eingabenettoströmung des spezifischen Gases in der Spülgaseinlassleitung (33a),
- Bestimmen, basierend auf dem Ausgabeanteil des spezifischen Gases in dem Ausgabespülgas und der Strömungsrate des Ausgabespülgases, einer Nettoströmung des spezifischen Gases in der Spülgasauslassleitung (33b) und
- Bestimmen einer Änderung in der Nettoströmung des spezifischen Gases an dem ersten Gasaustauschpunkt (23) basierend auf der Eingabenettoströmung und der Ausgabenettoströmung des spezifischen Gases, wobei die Änderung in der Nettoströmung einen Gasaustausch des spezifischen Gases an dem ersten Gasaustauschpunkt (3) angibt.

4. System (1) nach einem der vorhergehenden Ansprüche, wobei der mindestens eine Prozessor (37) zu Folgendem konfiguriert ist:

- Bestimmen eines Eingabeanteils des spezifischen Gases in dem Atemgas basierend auf der Analyse durch den Gasanalysator (105) der Gasproben aus der Inspirationsleitung (11),
- Bestimmen eines Ausgabeanteils des spezifischen Gases in dem Ausatemgas basierend auf der Analyse durch den Gasanalysator (105) der Gasproben aus der Exspirationsleitung (13) und
- Bestimmen, basierend auf dem Eingabe- und dem Ausgabeanteil des spezifischen Gases, eines Maßes an Gasaustausch des spezifischen Gases an dem zweiten Gasaustauschpunkt (3).

5. System (1) nach Anspruch 4, wobei der mindestens eine Prozessor (37) zu Folgendem konfiguriert ist:

- Empfangen einer Messung einer Strömungsrate [$V_{in}$] des Atemgases in der Inspirationsleitung (11),
- Empfangen einer Messung einer Strömungsrate [$V_{out}$] des Ausatemgases in der Exspirationsleitung (13),
- Bestimmen, basierend auf dem Eingabeanteil des spezifischen Gases in dem Atemgas und der Strömungsrate des Atemgases, einer Eingabenettoströmung des spezifischen Gases in der Inspirationsleitung (11),
- Bestimmen, basierend auf dem Ausgabeanteil des spezifischen Gases in dem Ausatemgas und der Strömungsrate des Ausatemgases, einer Nettoströmung des spezifischen Gases in der Exspirationsleitung (13) und
- Bestimmen einer Änderung in der Nettoströmung des spezifischen Gases an dem ersten Gasaustauschpunkt (3) basierend auf der Eingabenettoströmung und der Ausgabenettoströmung des spezifischen Gases, wobei die Änderung in der Nettoströmung einen Gasaustausch des spezifischen Gases an dem zweiten Gasaustauschpunkt (3) angibt.

6. System (1) nach einem der vorhergehenden Ansprüche, wobei das System (1) einen Gasprobenaufbereiter zum Entfernen von Wasserdampf in den Gasproben aus der Spülgaseinlassleitung (33a) und/oder den Gasproben aus der Spülgasauslassleitung (33b) vor der Analyse der Gasproben durch den Gasanalysator (105) umfasst, wobei der mindestens eine Prozessor (37) zu Folgendem konfiguriert ist:

- Bestimmen des Eingabeanteils des spezifischen Gases in dem Eingabespülgas basierend auf einem Anteil des spezifischen Gases in einer analysierten Eingabespülgasprobe und einem geschätzten Entfernen von Wasserdampf [$\Delta FH2O_{in}$] aus der Eingabespülgasprobe durch den Gasprobenaufbereiter und/oder
- Bestimmen des Ausgabeanteils des spezifischen Gases in dem Ausgabespülgas basierend auf einem Anteil des spezifischen Gases in einer analysierten Ausgabespülgasprobe und einem geschätzten Entfernen von Wasserdampf [$\Delta FH2O_{out}$] aus der Ausgabespülgasprobe durch den Gasprobenaufbereiter.

7. System (1) nach einem der vorhergehenden Ansprüche, wobei das System (1) einen Gasprobenaufbereiter zum Entfernen von Wasserdampf in den Gasproben aus der Inspirationsleitung (11) und/oder den Gasproben aus der Exspirationsleitung (13) vor der Analyse der Gasproben durch den Gasanalysator (105) umfasst, wobei der mindestens eine Prozessor (37) zu Folgendem konfiguriert ist:

- Bestimmen des Eingabeanteils des spezifischen Gases in dem Atemgas basierend auf einem Anteil des spezifischen Gases in einer analysierten Atemgasprobe und einem geschätzten Entfernen von Wasserdampf [$\Delta FH2O_{in}$] aus der Atemgasprobe durch den Gasprobenaufbereiter und/oder

- Bestimmen des Ausgabeanteils des spezifischen Gases in dem Ausatemgas basierend auf einem Anteil des spezifischen Gases in einer analysierten Ausatemgasprobe und einem geschätzten Entfernen von Wasserdampf [$\Delta FH2O_{out}$] aus der Ausatemgasprobe durch den Gasprobenaufbereiter.

8. System (1) nach einem der vorhergehenden Ansprüche, wobei der Gasanalysator (105) zudem mit einer Kalibrierungsgasquelle (110) zur Abgabe eines Kalibrierungsgases verbunden ist, wobei der Gasanalysator (105) dazu konfiguriert ist, aufeinanderfolgend Gasproben aus der Spülgaseinlassleitung (33a), der Spülgasauslassleitung (33b), der Inspirationsleitung (11), der Exspirationsleitung (13) und der Kalibrierungsgasquelle (110) aufzunehmen und zu analysieren.

9. System (1) nach einem der vorhergehenden Ansprüche, wobei das spezifische Gas ausgewählt ist aus der Gruppe bestehend aus Kohlenstoffdioxid [CO2] und Sauerstoff [O2].

10. System (1) nach einem der vorhergehenden Ansprüche, wobei der Gasanalysator (105) einen ersten Sensor zum Messen eines Anteils von CO2 [$FCO2_{in}$] in dem Eingabespülgas und dem Inspirationsgas und eines Anteils von CO2 [$FCO2_{out}$] in dem Ausgabespülgas und dem Ausatemgas und einen zweiten Sensor zum Messen eines Anteils von O2 [$FO2_{in}$] in dem Eingabespülgas und dem Inspirationsgas und eines Anteils von O2 [$FO2_{out}$] in dem Ausgabespülgas und dem Ausatemgas umfasst.

11. System (1) nach Anspruch 10, wobei der erste Sensor ein nicht dispersiver Infrarot-CO2-Sensor, [NDIR]-Sensor, ist und der zweite Sensor ein paramagnetischer oder elektrochemischer O2-Sensor ist.

12. System (1) nach einem der vorhergehenden Ansprüche, wobei das System (1) dazu konfiguriert ist, Gasaustauschinformationen auf einem Monitor (115) des Systems (1) anzuzeigen, wobei die Gasaustauschinformationen Informationen in Bezug auf das Maß an Gasaustausch des spezifischen Gases an dem ersten Gasaustauschpunkt (23), Informationen in Bezug auf das Maß an Gasaustausch des spezifischen Gases an dem zweiten Gasaustauschpunkt (3) und/oder Informationen in Bezug auf ein Maß an Gesamtgasaustausch des spezifischen Gases an dem ersten und dem zweiten Gasaustauschpunkt (3, 23) umfassen.

## Revendications

1. Système (1) d'analyse de concentrations de gaz avant et après un point d'échanges gazeux (3, 23), le système (1) comprenant :

un dispositif (5) pour l'échange gazeux sanguin extracorporel, le dispositif (5) comprenant :

- un oxygénateur (21) incluant une membrane (23) agissant comme une barrière gaz-liquide permettant le transfert de CO2 entre un flux sanguin et un flux de gaz de balayage à travers l'oxygénateur, dans lequel la membrane (23) constitue un premier point d'échanges gazeux, et
- une ligne d'entrée de gaz de balayage (33a) pour transporter un gaz de balayage d'entrée vers la membrane (23), et une ligne de sortie de gaz de balayage (33b) pour transporter un gaz de balayage de sortie à partir de la membrane (23), et

un ventilateur mécanique (7) pour ventiler mécaniquement un patient (3) par la fourniture de gaz respiratoire aux poumons du patient (3), dans lequel les poumons du patient (3) constituent un second point d'échanges gazeux, le ventilateur (7) comprenant :

- une ligne inspiratoire (11) pour transporter un flux de gaz respiratoire vers le patient (3), et une ligne expiratoire (13) pour transporter un flux de gaz d'expiration à partir du patient (3),

**caractérisé en ce qu'**un

analyseur de gaz (105) est relié à la fois à la ligne d'entrée de gaz de balayage (33a), à la ligne de sortie de gaz de balayage (33b), à la ligne inspiratoire (11) et à la ligne expiratoire (13) et est configuré pour recevoir et analyser de manière séquentielle des échantillons de gaz provenant de la ligne d'entrée de gaz de balayage (33a), de la ligne de sortie de gaz de balayage (33b), de la ligne inspiratoire (11) et de la ligne expiratoire (13), l'analyseur de gaz comprenant l'au moins un processeur (37) configuré pour déterminer une mesure d'échanges gazeux du gaz spécifique au niveau du premier point d'échanges gazeux sur la base de l'analyse des échantillons de gaz provenant de la ligne d'entrée de gaz de balayage (33a) et de la ligne de sortie de gaz de balayage (33b), et pour déterminer une mesure d'échanges gazeux du gaz spécifique au niveau du second point d'échanges gazeux sur la base de l'analyse des échantillons de gaz provenant de la ligne inspiratoire (11) et de la ligne expiratoire (13).

2. Système (100 ; 1) selon la revendication 1, dans lequel l'au moins un processeur (37) est configuré pour :

   - déterminer une fraction d'entrée du gaz spécifique dans le gaz de balayage d'entrée sur la base de l'analyse par l'analyseur de gaz (105) des échantillons de gaz provenant de la ligne d'entrée de gaz de balayage (33a),
   - déterminer une fraction de sortie du gaz spécifique dans le gaz de balayage de sortie sur la base de l'analyse par l'analyseur de gaz (105) des échantillons de gaz provenant de la ligne de sortie de gaz de balayage (33b), et
   - déterminer, sur la base des fractions d'entrée et de sortie du gaz spécifique, une mesure d'échanges gazeux du gaz spécifique au niveau du premier point d'échanges gazeux (23).

3. Système (1) selon la revendication 2, dans lequel l'au moins un processeur (37) est configuré pour :

   - recevoir une mesure d'un débit [$\dot{V}_{in}$] du gaz de balayage d'entrée dans la ligne d'entrée de gaz de balayage (33a),
   - recevoir une mesure d'un débit [$V_{out}$] du gaz de balayage de sortie dans la ligne de sortie de gaz de balayage (33b),
   - déterminer, sur la base de la fraction d'entrée du gaz spécifique dans le gaz de balayage d'entrée et du débit du gaz de balayage d'entrée, un flux net d'entrée du gaz spécifique dans la ligne d'entrée de gaz de balayage (33a),
   - déterminer, sur la base de la fraction de sortie du gaz spécifique dans le gaz de balayage de sortie et du débit du gaz de balayage de sortie, un flux net du gaz spécifique dans la ligne de sortie de gaz de balayage (33b), et
   - déterminer un changement de flux net du gaz spécifique au niveau du premier point d'échanges gazeux (23) sur la base du flux net d'entrée et du flux net de sortie du gaz spécifique, lequel changement de flux net est indicatif d'un échange gazeux du gaz spécifique au niveau du premier point d'échanges gazeux (3).

4. Système (1) selon l'une quelconque des revendications précédentes, dans lequel l'au moins un processeur (37) est configuré pour :

   - déterminer une fraction d'entrée du gaz spécifique dans le gaz respiratoire sur la base de l'analyse par l'analyseur de gaz (105) des échantillons de gaz provenant de la ligne inspiratoire (11),
   - déterminer une fraction de sortie du gaz spécifique dans le gaz d'expiration sur la base de l'analyse par l'analyseur de gaz (105) des échantillons de gaz provenant de la ligne expiratoire (13), et
   - déterminer, sur la base des fractions d'entrée et de sortie du gaz spécifique, une mesure d'échanges gazeux du gaz spécifique au niveau du second point d'échanges gazeux (3).

5. Système (1) selon la revendication 4, dans lequel l'au moins un processeur (37) est configuré pour :

   - recevoir une mesure d'un débit [$V_{in}$] du gaz respiratoire dans la ligne inspiratoire (11),
   - recevoir une mesure d'un débit [$V_{out}$] du gaz d'expiration dans la ligne expiratoire (13),
   - déterminer, sur la base de la fraction d'entrée du gaz spécifique dans le gaz respiratoire et du débit du gaz respiratoire, un flux net d'entrée du gaz spécifique dans la ligne inspiratoire (11),
   - déterminer, sur la base de la fraction de sortie du gaz spécifique dans le gaz d'expiration et du débit du gaz d'expiration, un flux net du gaz spécifique dans la ligne expiratoire (13), et
   - déterminer un changement de flux net du gaz spécifique au niveau du premier point d'échanges gazeux (3) sur la base du flux net d'entrée et du flux net de sortie du gaz spécifique, lequel changement de flux net est indicatif d'un échange gazeux du gaz spécifique au niveau du second point d'échanges gazeux (3).

6. Système (1) selon l'une quelconque des revendications précédentes, le système (1) comprenant un conditionneur d'échantillon de gaz pour l'élimination de vapeur d'eau dans les échantillons de gaz provenant de la ligne d'entrée de gaz de balayage (33a) et/ou les échantillons de gaz provenant de la ligne de sortie de gaz de balayage (33b), avant l'analyse des échantillons de gaz par l'analyseur de gaz (105), l'au moins un processeur (37) étant configuré pour :

   - déterminer la fraction d'entrée du gaz spécifique dans le gaz de balayage d'entrée sur la base d'une fraction du gaz spécifique dans un échantillon de gaz de balayage d'entrée analysé et d'une élimination estimée de vapeur

d'eau [$\Delta FH2O_{in}$] de l'échantillon de gaz de balayage d'entrée par le conditionneur d'échantillon de gaz, et/ou
- déterminer la fraction de sortie du gaz spécifique dans le gaz de balayage de sortie sur la base d'une fraction du gaz spécifique dans un échantillon de gaz de balayage de sortie analysé et d'une élimination estimée de vapeur d'eau [$\Delta FH2O_{out}$] de l'échantillon de gaz de balayage de sortie par le conditionneur d'échantillon de gaz.

7. Système (1) selon l'une quelconque des revendications précédentes, le système (1) comprenant un conditionneur d'échantillon de gaz pour l'élimination de vapeur d'eau dans les échantillons de gaz provenant de la ligne inspiratoire (11) et/ou les échantillons de gaz provenant de la ligne expiratoire (13), avant l'analyse des échantillons de gaz par l'analyseur de gaz (105), l'au moins un processeur (37) étant configuré pour :

   - déterminer la fraction d'entrée du gaz spécifique dans le gaz respiratoire sur la base d'une fraction du gaz spécifique dans un échantillon de gaz respiratoire analysé et d'une élimination estimée de vapeur d'eau [$\Delta FH2O_{in}$] de l'échantillon de gaz respiratoire par le conditionneur d'échantillon de gaz, et/ou
   - déterminer la fraction de sortie du gaz spécifique dans le gaz d'expiration sur la base d'une fraction du gaz spécifique dans un échantillon de gaz d'expiration analysé et d'une élimination estimée de vapeur d'eau [$\Delta FH2O_{out}$] de l'échantillon de gaz d'expiration par le conditionneur d'échantillon de gaz.

8. Système (1) selon l'une quelconque des revendications précédentes, dans lequel l'analyseur de gaz (105) est relié également à une source de gaz d'étalonnage (110) pour la distribution d'un gaz d'étalonnage, l'analyseur de gaz (105) étant configuré pour recevoir et analyser de manière séquentielle des échantillons de gaz provenant de la ligne d'entrée de gaz de balayage (33a), de la ligne de sortie de gaz de balayage (33b), de la ligne inspiratoire (11), de la ligne expiratoire (13) et de la source de gaz d'étalonnage (110).

9. Système (1) selon l'une quelconque des revendications précédentes, dans lequel le gaz spécifique est choisi dans le groupe constitué par le dioxyde de carbone [CO2] et l'oxygène [02].

10. Système (1) selon l'une quelconque des revendications précédentes, dans lequel l'analyseur de gaz (105) comprend un premier capteur pour mesurer une fraction de CO2 [$FCO2_{in}$] dans le gaz de balayage d'entrée et le gaz inspiratoire et une fraction de CO2 [$FCO2_{out}$] dans le gaz de balayage de sortie et le gaz d'expiration, et un second capteur pour mesurer une fraction d'O2 [$FO2_{in}$] dans le gaz de balayage d'entrée et le gaz inspiratoire et une fraction d'O2 [$FO2_{out}$] dans le gaz de balayage de sortie et le gaz d'expiration.

11. Système (1) selon la revendication 10, dans lequel le premier capteur est un capteur de CO2 infrarouge non dispersif [NDIR] et le second capteur est un capteur d'O2 paramagnétique ou électrochimique.

12. Système (1) selon l'une quelconque des revendications précédentes, le système (1) étant configuré pour afficher des informations d'échanges gazeux sur un moniteur (115) du système (1), les informations d'échanges gazeux comprenant des informations relatives à la mesure d'échanges gazeux du gaz spécifique au niveau du premier point d'échanges gazeux (23), des informations relatives à la mesure d'échanges gazeux du gaz spécifique au niveau du second point d'échanges gazeux (3), et/ou des informations relatives à une mesure d'échanges gazeux totale du gaz spécifique au niveau du premier et du second points d'échanges gazeux (3, 23).

Fig. 1

Fig. 2

Fig. 3

Fig. 4

Adding CO2 to the sweep gas flow upstream of the oxygenator to control a degree of CO2 removal from the bloodstream by the oxygenator — S11

S12

Measuring fraction of CO2 [$FCO2_{in}$] in input sweep gas flow — S12a

Measuring input sweep gas flow rate ($\dot{V}_{in}$) — S12b

Measuring fraction of CO2 [$FCO2_{out}$] in output sweep gas flow — S12c

Measuring output sweep gas flow rate ($\dot{V}_{out}$) — S12d

Calculating net CO2 exchange [$\dot{V}CO2_{net}$] over membrane based on measured $FCO2_{in}$, $\dot{V}_{in}$, $FCO2_{out}$ and $\dot{V}_{out}$ — S12e

Utilizing $\dot{V}CO2_{net}$ as a measure of CO2 removal for improved regulation of the CO2 addition to the sweep gas flow — S13

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021353892 A
- US 2020359935 A
- US 2014216252 A